# EUROPEAN PATENT SPECIFICATION

(11) **EP 3 252 031 B1**
(45) Date of publication and mention of the grant of the patent: **08.05.2024**
(21) Application number: 17160213.9
(22) Date of filing: 09.03.2017
(51) Int. Cl.: C07C 29/74

(54) **A METHOD FOR PURIFICATION OF ORGANIC WATER-SOLUBLE COMPOUNDS FROM HYDROPHOBIC CONTAMINANTS**
VERFAHREN ZUR REINIGUNG VON ORGANISCHEN WASSERLÖSLICHEN VERBINDUNGEN AUS HYDROPHOBEN VERUNREINIGUNGEN
PROCÉDÉ DE PURIFICATION DE COMPOSÉS HYDROSOLUBLES ORGANIQUES À PARTIR DE CONTAMINANTS HYDROPHOBES

(30) Priority: 15.03.2016 SK 500152016
(43) Date of publication of application: 06.12.2017
(73) Proprietor: Ústav experimentálnej fyziky Slovenskej Akadémie vied, verejná vyskumná institúcia, 040 01 Kosice (SK)
(72) Inventor: Sedlák, Marián, 040 01 Kosice (SK); Rak, Dmytro, 040 23 Kosice (SK)
(74) Representative: Holoubková, Mária

(56) References cited:
- WO-A2-2015/105463
- MO ET AL: "Treatment of dye aqueous solutions using nanofiltration polyamide composite membranes for the dye wastewater reuse", DYES AND PIGMENTS, ELSEVIER APPLIED SCIENCE PUBLISHERS. BARKING, GB, vol. 76, no. 2, 14 September 2007 (2007-09-14), pages 429-434, XP022245326, ISSN: 0143-7208, DOI: 10.1016/J.DYEPIG.2006.09.007

## Description

### Engineering Area

The invention concerns a method for purification of organic water-soluble compounds from hydrophobic contaminants using nanosegregation in aqueous solutions.

### Current State of the Art

The need for pure to very pure organic water-soluble compounds arises from their use for example in food industry, pharmaceutical industry or in research where is a strong need for high-purity solvents for analysis, for standards, pure compounds for experiments, synthesis, etc. An important group of contaminants is represented by hydrophobic contaminants covering e.g. higher alkanes (paraffins) present in lubricants used in industrial facilities, phthalates that come from the contact of organic compounds with plastic containers and caps, polycyclic aromatic hydrocarbons (PAHs) - dioxins and polychlorinated biphenyls (PCBs) which are listed in the Commission Regulation (EC) No 1881/2006 (separately Commission Regulation (EU) No 835/2011 for PAHs) as substances controlled in certain types of foodstuffs with a maximum level not exceeding 1-35 ppm, depending on the foodstuff [Commission Regulation (EC) No 1881/2006 of 19 December 2006 setting maximum levels for certain contaminants in foodstuffs; Commission Regulation (EU) No. 835/2011 of 19 August 2011 amending Regulation (EC) No 1881/2006 regarding maximum levels for polycyclic aromatic hydrocarbons in foodstuffs]. Other hydrophobic compounds such as phthalates, alkanes and their derivatives, hydrophobic aromatic compounds, etc. are regulated by the Commission Directive 2002/72/EC as compounds that can be transferred into foodstuffs from materials intended for contact with foodstuffs (plastic materials and articles) [Commission Directive 2002/72/EC of 6 August 2002 relating to plastic materials and articles intended to come into contact with foodstuffs]. Minimization of amount of these contaminants in liquids used in the production of food and beverages (such as glycerol, ethanol, etc.) and extraction solvents used in the production of food and food ingredients (such as acetone, ethanol, methanol, isopropanol, etc.), is very important.

Currently, there is no method specially intended for the purification of water-soluble organic compounds from hydrophobic contaminants. Purification of water-soluble organic compounds is carried out by common methods, not specifically aimed at the removal of hydrophobic contaminants, which include for example distillation, recrystallization, preparative chromatography, extraction, and some other less frequently used methods. The selection of a particular method depends on physical properties of the compound, volume (weight), desired product purity, price factor, etc.

Distillation (and also rectification) is particularly suitable for the separation of liquids with significantly different boiling points, i.e. for separation of liquid contaminants with boiling points significantly different from that of the purified liquid. The advantages of this method are relatively simple equipment, possibility of purification of large volumes of liquids, and low operational costs. The disadvantages of distillation include inapplicability for separation of compounds with approximately the same boiling points and impossibility of separation of two- and multi-component azeotropic mixtures, i.e. impossibility of removing contaminants which form azeotropic mixtures with the purified compound. Another disadvantage of distillation is its inapplicability for most solid compounds.

Recrystallization (often in combination with filtration) is a simple method used in particular for the purification of solid compounds from impurities of different nature. The disadvantages of recrystallization are: the need for large volumes of pure and expensive solvents, impossibility or difficulty to use it for liquids, and hazardous character of certain solvents.

Extraction is a commonly used purification method of compounds which allows the separation of compounds based on their different solubility in immiscible solvents. The disadvantages are the need for pure, expensive as well as often harmful solvents, relatively small volumes (weights) of compounds, and relatively high operational costs.

Preparative chromatography is a common method used for the purification of organic compounds which provides a very efficient separation of individual compounds. The disadvantages, however, are the high cost of equipment, possibility of separation of only relatively small volumes (weights) of compounds, expensive operation (the need for exchange of mobile and stationary phase) and the need for highly qualified staff to operate the relatively sophisticated and expensive equipment.

Another alternative method is molecular filtration based on the separation of compounds based on differing molecular dimensions of the compounds to be separated described for instance in document Mo et al: "Treatment of dye aqueous solutions using nanofiltration polyamide polyamide composite membranes for the dye wastewater reuse", DYES AND PIGMENTS, ELSEVIER APPLIED SCIENCE PUBLISHERS, BARKING, GB, vol. 76, no. 2, 14 September 2007, pages 429-434. Filtration is performed through a membrane with molecular weight cut-off (MWCO) smaller than the molecular weight of the impurity to be removed. The purified compound and the contaminant must therefore differ sufficiently in molecular weight. This method also requires substantially high filtration pressures.

In certain cases, purification of organic compounds can be achieved by phase separation of the unwanted impurity. Such separation can be achieved by altering pressure-temperature conditions or mixing the organic compound with some additives aimed at getting the system to the point where the organic compound of interest becomes immiscible with the impurity (distinct macrophases are formed). The phase that contains the unwanted impurity can be then mechanically removed from the system. Several problems are, however, involved such as the fact that the impurity does not have to be distributed solely in one of the phases or that the macrophases cannot be fully mechanically separated from each other which then leads only to a gross purification, not to the purification to high degrees of purity.

None of the above mentioned methods is directly aimed at the purification of organic water-soluble compounds from hydrophobic impurities, even if the correct choice of method or combination of methods can lead to the elimination of hydrophobic contaminants content in the purified compound. In contrast to the above described methods, the present method according to this invention is focused on the removal of hydrophobic compounds and does not suffer from the above mentioned shortcomings and problems.

### Summary of the Invention

The principle of the present invention is the purification method of organic water-soluble compounds from hydrophobic contaminants, which consists in mixing an organic water-soluble compound containing hydrophobic contaminants with water in a ratio which results in nanosegregation of hydrophobic compounds (contaminants) contained in the organic compound into nanoparticles, in contrast with the existing general idea, that hydrophobic compounds initially contained in the organic water-soluble compound when mixed with water remain up to a certain critical concentration of hydrophobic compounds molecularly dissolved and above this critical concentration segregate into a macrophase (thus creating a macroscopic phase - liquid continuum of macroscopic dimensions or solid phase of macroscopic dimensions).

The present purification method of organic water-soluble compounds with solubility in water at least 1% from hydrophobic contaminants consists in mixing of a liquid organic water-soluble compound with water in such a ratio that the weight content of the liquid organic water-soluble compound is from 1% to 50%, preferably from 10% to 25%. In the case of solid organic water-soluble compounds it is necessary to perform the mixing of the solid organic water-soluble compound with water such that the weight content of the solid organic water-soluble compound is from 1% to a value at which the given solid organic compound is still soluble in water, whereby it is preferred that the content of the solid compound containing hydrophobic contaminants in the mixture with water is the highest possible within the available solubility range for the given particular compound.

By means of the above described method, nanosegregation of hydrophobic compounds (contaminants) contained in the organic compound into nanoparticles occurs, whereby it was found that the formed nanoparticles:
a) are stable over time
b) fill evenly the entire volume of the mixture
c) are mechanically substantially strong and stable
d) are relatively stable in shape
e) are formed at concentrations of contaminants in organic water-soluble compounds in a very wide range (concentration range of contaminants of several orders, typically from 0.0001% to 0.2%)
f) contain virtually the entire contaminant originally contained in the purified compound (in other words the concentration of contaminants in the mixture outside the nanoparticles is practically zero)
g) their size is of the order of about 100 nm.

Based on these findings, a subsequent step of the presented purification method of organic water-soluble compounds from hydrophobic contaminants is proposed according to the invention: nanofiltration of the mixture prepared in the first step, adjusted to the above mentioned parameters of the nanoparticles, in particular by appropriate selection of the effective pore size and filtration pressures. Due to the above mentioned characteristics of nanosegregation and the adjusted parameters of nanofiltration, virtually a full removal of nanoparticles is achieved, which also means a virtually full removal of hydrophobic contaminants. The content of hydrophobic contaminants in the water-soluble organic compound can be reduced to below 0.5 ppm after purification by the presented method. The essential parameters of nanofiltration are the so called effective pore size, filtration pressure, and chemical compatibility (chemical resistance) of the material from which the filter is made in regard to the nature of the purified compound and contaminants. In regard to the nano dimensions of the segregated particles it is necessary to choose filters with effective pore sizes also in the nano region, because conventional filters with order of magnitude larger pores, applicable for filtration of precipitates of macroscopic dimensions during macrophase separation of solid compounds, would be non-functional. In the case of segregation of liquid contaminants, the nano dimensions of segregated particles are again crucial, because during macrophase separation it is not possible to separate the macrophases by means of any filtration method.

Considering the above mentioned circumstances, chemically resistant nanoporous filters with an effective pore size of 5 nm - 500 nm and filtration pressures from 10.1 kPa (0.1 atm) to 1.01 MPa (10 atm) were used, which allow the removal of hydrophobic contaminants segregated into nanoparticles/nanodroplets without undesired disintegration or shape deformation of the nanoparticles/nanodroplets allowing passing through the pores of the filter, and without the necessity of addition of surfactants as stabilizers of the nanoparticles/nanodroplets and a subsequent contamination of the mixture with surfactants. The preferred pore size of the nanoporous filter is from 100 nm to 300 nm and the preferred filtration pressure is from 50.7 kPa (0.5 atm) to 304.0 kPa (3 atm).

The final step of the method according to this invention is the subsequent separation of water from the purified compound by evaporation or distillation.

The present patent application is loosely related to our previous application (document WO 2015/105463 A2) disclosing that mixing of organic water-miscible liquids (not solids) with water leads under specific conditions to the segregation of hydrophobic contaminants into nanoobjects instead of forming a macroscopic phase and that such nanoscopic segregation can be quantified in such a way that gives a quantitative correlation with the content of hydrophobic contaminants. The previous invention, alone or in combination with any other document(s), however does not teach anything about whether (and at which conditions or which preparation routes) such or similar nanoobjects are sufficiently mechanically firm and stable in shape to be subsequently removable by filtration. Especially without necessity of addition of any additives as stabilizers of nanoparticles/nanodroplets and subsequent secondary contamination of the mixture with these additives. The prior art, as a whole, oppositely prompts any skilled person to expect that stabilizers such as surfactants are inevitable, otherwise extremely high surface tension at the nanodroplets would result in their coalescence and impossibility of their removal by filtration. It is highly non-obvious that such nanoobjects do not coalesce under filtration conditions. We have discovered that this functioning of nanodroplets is due to their substantial negative surface zeta potential (electrical double layer). This potential is a key to overcoming the technical difficulty in completing the given task (purification). This potential is conditional on the preparation route and parameters of the preparation procedure defined strictly in the patent claims.

The purification method according to this invention can be used for a wide range of total content of contaminants in the organic water-soluble compound. This range is from 0.0005% to 0.1 wt. % for the vast majority of organic water-soluble compounds. For a narrower class of organic water-soluble compounds, this range is from 0.0001% to 0.2 wt. %.

The effect of the purification method according to this invention is such that the content of the hydrophobic contaminant in the water-soluble organic compound is reduced after purification to a value of less than 5.0 ppm, or to a value of less than 0.5 ppm.

An essential feature of the purification method according to this invention is the fact that it consists of combination of steps leading to nanosegregation and subsequent setting of filtration parameters based on the knowledge of the properties and characteristics of the nanoparticles and of the entire process of nanosegregation.

An essential feature of the method according to this invention is also the fact that the separation of contaminants into discrete, relatively stable and solid (subsequently filterable) nanoparticles occurs after the addition of water only, without need of the addition of any chemicals (stabilizers, surfactants) which could present a secondary contamination and would also increase the cost of purification.

The method of purification according to this invention can be applied to virtually any organic water-soluble compound with solubility in water at least 1%. Examples presented include liquids such as alcohols (ethanol, tert-butyl alcohol), acetone, tetrahydrofuran, dimethoxyethane, acids such as acetic acid, formic acid; ionic and non-ionic solid crystalline compounds such as urea, glucose, citric acid, etc.

Hydrophobic contaminants are alkanes such as decane, dodecane, tetradecane, hexadecane and octadecane; polychlorinated biphenyls such as polychlorinated biphenyl 14; polycyclic aromatic hydrocarbons such as benzo(a)pyrene, naphthol, biphenyl; phthalates such as diethylhexyl phthalate.

Nanosegregation takes place in a temperature range in which the mixture of organic water-soluble compound with water is in the liquid phase, whereby for particular compounds as well as for different values of content of the organic compound in the mixture with water, the preferred range of temperature values can differ.

The method according to this invention is applicable for the weight content of the organic water-soluble liquid compound in the mixture with water in the range from 1% to 50%, since the effect of nanosegregation and subsequent filterability of nanoparticles/nanodroplets are feasible in this interval. This interval can be further preferably narrowed to the range from 3% to 30%, more preferably to the range from 10% to 25%. This interval optimization is based on several factors. Certain extremely small amount of the contaminant remains molecularly dissolved also in the aqueous mixture, and thus remains unfilterable. The smaller the ratio of the water-soluble organic compound in the mixture with water is, the smaller this amount is. In order to optimize the extremely low residual contamination after purification it is the preferred to carry out the purification process at higher ratios of water in the mixture. In order to optimize the process economics it is preferred to carry out the purification process at lower ratios of water in the mixture, as it minimizes the costs for water as a material and particularly for the back-removal of water from the mixture after filtration. The combination of both factors leads to the optimization/narrowing of the suitable range, from 1% to 50%, towards the mean of this range (from 3% to 30%, more preferably from 10% to 25%). In addition to these two factors, the interval from 10% to 25% is preferred also in view of the fact that the time stability of the nanoparticles/nanodroplets is maximal in this interval.

Compared to the so-far used methods, the method according to this invention is advantageous in view of the following facts:
- the only medium which is used in the purification method according to this invention is water, which is a cheap and harmless compound
- volumes (weights) of purified compounds are not limited
- price of equipment (technology) is low
- operational costs are low
- highly skilled employees are not required.

### Summary of drawings / figures

Fig. 1 shows the angular dependence of intensity of laser light scattered from nanoparticles before filtration and after filtration by nanoporous filter in the process of purification of acetone.
Fig. 2 shows comparison of contaminants content in acetone before filtration and after filtration of the mixture by nanoporous filter, respectively. Obtained by GC-MS HS-SPME method.
Fig. 3 shows the dependence of the decrease of signal intensity of the mass spectrometer detector after filtration for individual contaminants marked 1 - 8 in Fig.2.
Fig. 4 shows the angular dependence of intensity of laser light scattered from nanoparticles before filtration and after filtration by nanoporous filter in the process of purification of acetic acid.
Fig. 5 shows the comparison of contaminants content in acetic acid before filtration and after filtration of the mixture by nanoporous filter, respectively. Obtained by GC-MS HS-SPME method.
Fig. 6 shows the angular dependence of intensity of laser light scattered from nanoparticles before filtration and after filtration by nanoporous filter in the process of purification of tert-butyl alcohol (TBA).
Fig. 7 shows the comparison of contaminants content in TBA before filtration and after filtration of the mixture by nanoporous filter, respectively. Obtained by GC-MS HS-SPME method.
Fig. 8 shows the dependence of the decrease of signal intensity of the mass spectrometer detector after filtration for individual contaminants marked 1 - 21 in Fig.7.
Fig. 9 shows the angular dependence of intensity of laser light scattered from nanoparticles before filtration and after filtration by nanoporous filter in the process of purification of intentionally and in a controlled manner contaminated ethanol.
Fig. 10 shows the comparison of contaminants content in intentionally and controllably contaminated ethanol before filtration and after filtration of the mixture by nanoporous filter, respectively. Obtained by GC-MS HS-SPME method.
Fig. 11 shows the angular dependence of intensity of laser light scattered from nanoparticles before filtration and after filtration by nanoporous filter in the process of purification of intentionally and in a controlled manner contaminated tetrahydrofuran.
Fig. 12 shows the comparison of contaminants content in intentionally and controllably contaminated tetrahydrofuran before filtration and after filtration of the mixture by nanoporous filter, respectively. Obtained by GC-MS HS-SPME method. Fig. 13 shows the angular dependence of intensity of laser light scattered from nanoparticles before filtration and after filtration by nanoporous filter in the process of purification of intentionally and in a controlled manner contaminated dimethoxyethane.
Fig. 14 shows the comparison of contaminants content in intentionally and controllably contaminated dimethoxyethane before filtration and after filtration of the mixture by nanoporous filter, respectively. Obtained by GC-MS HS-SPME method.
Fig. 15 shows the angular dependence of intensity of laser light scattered from nanoparticles before filtration and after filtration by nanoporous filter in the process of purification of urea.
Fig. 16 shows the comparison of contaminants content in urea before filtration and after filtration of the mixture by nanoporous filter, respectively. Obtained by GC-MS HS-SPME method.
Fig. 17 shows by means of static laser light scattering measurements the presence of nanoparticles in TBA/water mixture and their removal by filtration through nanoporous filters of different effective pore diameters at a pressure of 507 kPa (5 atm).
Fig. 18 shows by means of static laser light scattering measurements the presence of nanoparticles in ethanol/water mixture and their removal by filtration through nanoporous filters with an effective pore diameter of 0.2 µm at different pressures.
Fig. 19 shows by means of static laser light scattering measurements the presence of nanoparticles in ethanol/water mixture and their removal by filtration through a nanoporous filter with an effective pore diameter of 0.2 µm at a pressure of 507 kPa (5 atm) for various levels of ethanol contamination with octadecane.
Fig. 20 shows the dependence of intensity of nanosegregation of a contaminant as a function of concentration of organic liquid (ethanol) in a mixture with water.
Fig. 21 shows by means of static laser light scattering measurements the presence of nanoparticles in ethanol/water mixture (2 wt. % solution of ethanol in water) and their removal by filtration through a nanoporous filter with an effective pore diameter of 0.2 µm at a pressure of 507 kPa (5 atm).
Fig. 22 shows by means of static laser light scattering measurements the presence of nanoparticles in ethanol/water mixture (50 wt. % solution of ethanol in water) and their removal by filtration through a nanoporous filter with an effective pore diameter of 0.2 µm at a pressure of 507 kPa (5 atm).

### Examples of Invention Embodiments

### Example 1

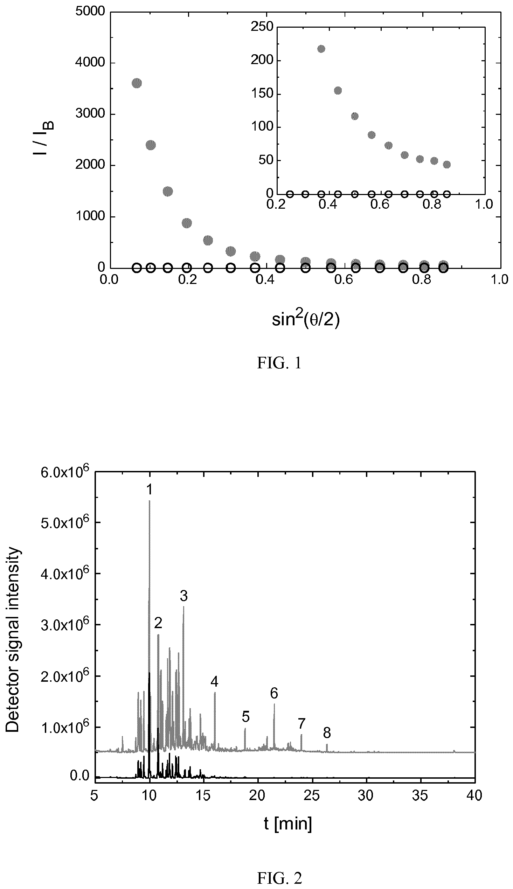

Acetone of a p.a. grade was mixed with water in a ratio of 1:4 (20 wt. % solution of acetone in water). This mixing caused generation of nanoparticles in solution formed by segregated hydrophobic contaminants contained in acetone. Their presence was quantified using the laser light scattering method (Fig. 1). The graph in Fig. 1 shows the angular dependence of the intensity I of scattered laser light from nanoparticles before filtration (gray solid circles) and after filtration by nanoporous filter (black open circles). Scattering intensities are normalized to the scattering intensity of a benzene standard I_{B}. The size of nanoparticles was determined from the angular dependence of scattered light as a size distribution ranging from R = 100 nm to R = 300 nm. θ is the scattering angle (the angle between the primary laser beam and the beam of the detected scattered light).

Contaminants were characterized in terms of chemical composition by means of GC-MS HS-SPME method (Gas Chromatography - Mass Spectrometry Head Space Solid-Phase Micro Extraction). Fig. 2 shows the results before filtration of the mixture (top gray curve) and after filtration of the mixture by nanoporous filter with an effective pore diameter of 0.2 µm at a filtration pressure of 507 kPa (5 atm), which removes the nanoparticles (bottom black curve). List of identified compounds: 1 - trimethylbenzene, 2 - limonene , 3 - undecane, 4 - dodecane, 5 - tridecane, 6 - tetradecane, 7 - pentadecane, 8 - hexadecane. t is the elution time. The detector signal intensity is in relative units. The elugrams show that the nanoparticles are composed primarily of hydrophobic compounds: undecane, dodecane, tridecane, tetradecane, pentadecane and hexadecane. The GC-MS elugram in Fig. 2 as well as all other elugrams in this application are presented after subtraction of the elugram of water (so called subtraction of a blank elugram). For the sake of clarity, the elugrams of filtered and unfiltered samples are mutually vertically displaced in all images in this application.

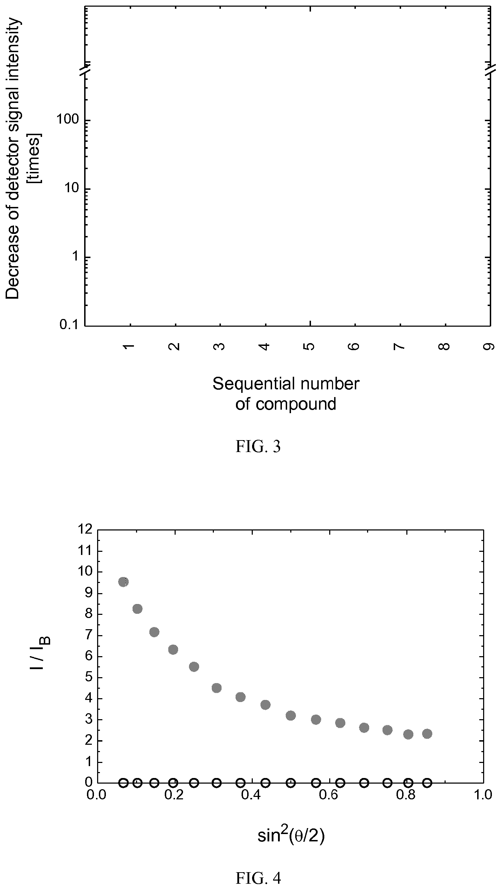

Fig. 3 shows the dependence of the decrease of signal intensity of the mass spectrometer detector after filtration for individual contaminants marked 1 - 8 in Fig. 2. The decrease of detector signal intensity after filtration corresponds to the degree of elimination of the particular contaminant. Sequential numbers correspond to the degree of hydrophobicity of the particular contaminant. Slightly hydrophobic compounds (1-2) are eliminated by a factor of 2-3, more hydrophobic compounds (3-8) are eliminated virtually completely (not measureable concentration after filtration).

After the above mentioned procedures acetone can be separated from water using commonly available separation methods (e.g. distillation) to obtain acetone of substantially higher purity, free of hydrophobic contaminants.

### Example 2

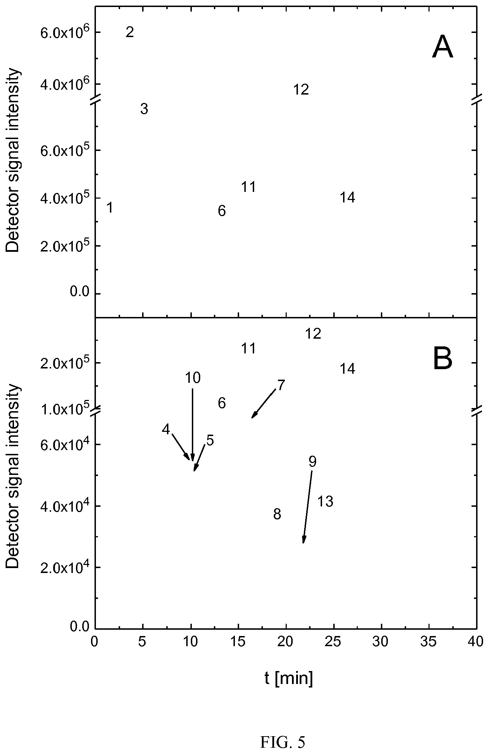

The procedure was the same as in Example 1. Acetic acid was used instead of acetone. Instead of the concentration of acetone in water of 20 wt. %., the concentration of acetic acid was 15 wt. %. Fig. 4 documents by means of static laser light scattering measurements the presence of nanoparticles in the acetic acid / water mixture and their removal by filtration through a nanoporous filter with an effective pore diameter of 0.2 µm at a filtration pressure of 507 kPa (5 atm). Fig. 5A shows results of measurements using GC-MS HS-SPME method focused on the chemical composition of the mixture before and after filtration, in particular on the presence of contaminants. Fig. 5B shows the detail of peaks with small amplitudes. List of identified compounds: 1 - ethanol, 2 - acetic acid , 3 - butyl acetate , 4 - butyl butyrate, 5 - octanal , 6 - nonanal , 7 - decanal , 8 - undecanal , 9 - dodecanal , 10 - decane , 11 - dodecane , 12 - tetradecane , 13 - pentadecane , 14 - hexadecane .

### Example 3

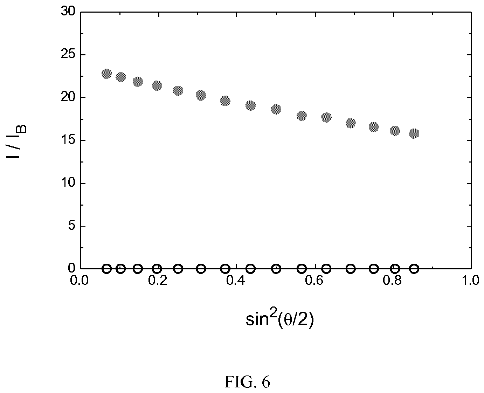

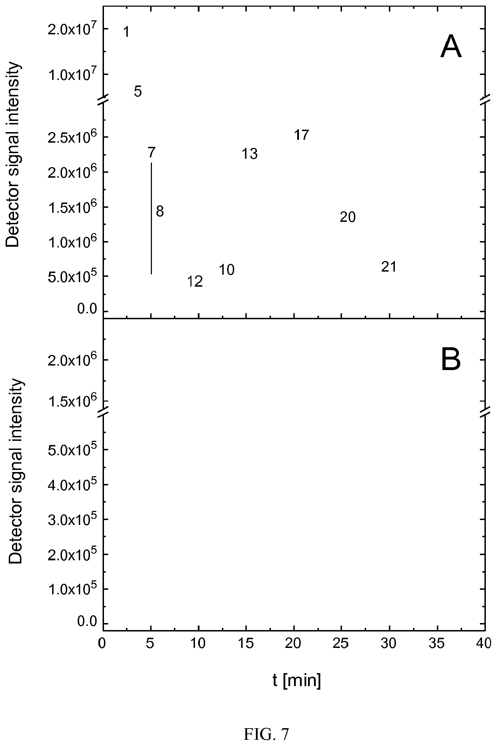

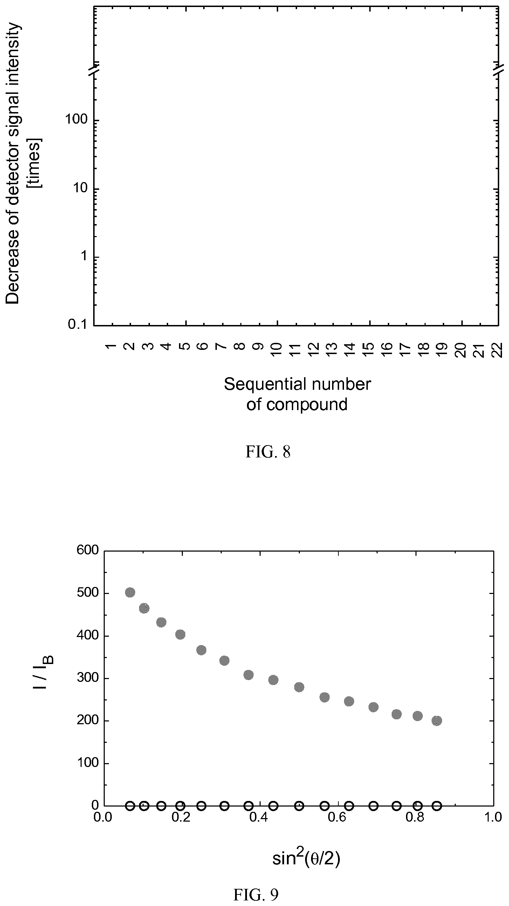

The procedure was the same as in Example 1. Tert-butyl alcohol (TBA) was used instead of acetone. Instead of the concentration of acetone in water of 20 wt. %., the concentration of TBA in water was 15 wt. %. Fig. 6 documents by means of static laser light scattering measurements the presence of nanoparticles in the TBA/water mixture and their removal by filtration through a nanoporous filter with an effective pore diameter of 0.1 µm at a filtration pressure of 507 kPa (5 atm). The size of nanoparticles was determined from the slope of angular dependence of scattered light as R= 58 nm (radius of nanoparticles), D = 116 nm (diameter of nanoparticles). Fig. 7 shows the results of measurements using GC-MS HS-SPME method focused on the chemical composition of the mixture before and after filtration, in particular on the presence of contaminants. (A) TBA (tert-butyl alcohol) in the mixture with water, concentration of TBA *c*_{TBA} = 150 g/kg, before filtration using nanoporous filter (top gray curve) and after filtration which removes the nanoparticles (bottom black curve). (B) detail showing peaks with small amplitudes. Identified compounds in the solution: 1 - tert-butyl alcohol, 2 - 2-butanol, 3 - di-tert-butyl ether, 4 - butanol, 5 - tert-butyl-2-butyl ether, 6 - butyl acetate, 7 - 1-(1,1-dimethylethoxy)-2,2-dimethylpropane, 8 - methylhexanol, 9 - 1-chloro-2-methylbenzene, 10 - decanal, 11 - 1-decanol, 12 - decane, 13 - dodecane, 14 - tridecane, 15 - dimethyldodecane, 16 - methyltridecane, 17 - tetradecane, 18 - dimethyltetradecane, 19 - methylpentadecane, 20 - hexadecane, 21 - octadecane. The elugrams show that the nanoparticles are composed primarily of hydrophobic compounds: dodecane, tetradecane, hexadecane and octadecane. Fig. 8 shows the dependence of the decrease of signal intensity of the mass spectrometer detector after filtration for individual contaminants marked 1 - 21 in Fig. 7. Sequential numbers correspond to the degree of hydrophobicity of the corresponding contaminant. Hydrophilic compounds are not eliminated by filtration (1-8), slightly hydrophobic compounds (9-10) are eliminated by a factor of cca 10, more hydrophobic compounds (11-13) are eliminated by a factor of cca 100, whereas highly hydrophobic compounds (14-21) are eliminated virtually completely (not measureable concentration after filtration).

### Example 4

Ethanol of p.a. grade was intentionally contaminated with the following contaminants: 1-hexanol (concentration 0.00150%), decane (concentration 0.00150%), octadecane (concentration 0.00150%), polychlorinated biphenyl PCB14 (concentration 0.00148%) and diethylhexyl phthalate (concentration 0.00152%). Overal concentration of contaminants 0.0075%. Added model contaminants were chosen according to their significance in terms of the potency of occurrence or in terms of health harmfulness.

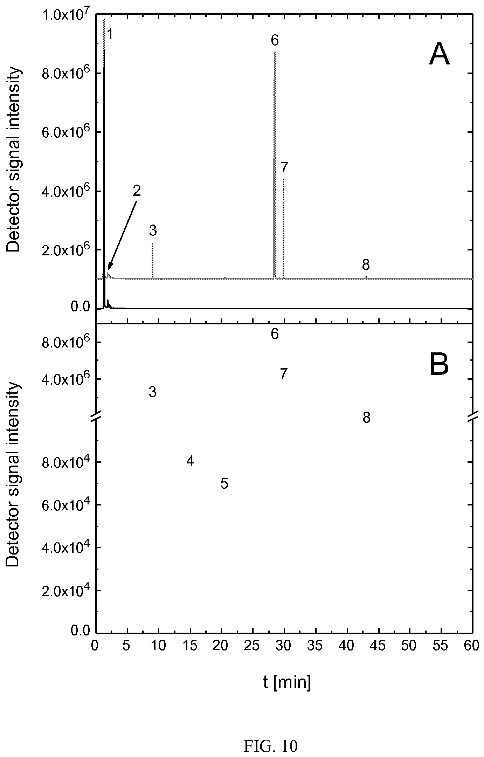

The contaminated ethanol was subsequently mixed with water in a ratio of 1:4 (20 wt. % solution of ethanol in water). Subsequently, the procedure was the same as in Example 1. Fig. 9 documents by means of static laser light scattering measurements the presence of nanoparticles in the contaminated ethanol/water mixture and their removal by filtration through a nanoporous filter with an effective pore diameter of 0.2 µm at a filtration pressure of 507 kPa (5 atm). Fig. 10 shows the results of measurements using GC-MS HS-SPME method focused on the chemical composition of the mixture before and after filtration, in particular on the presence of contaminants. List of identified compounds: 1 - ethanol, 2 - 1-hexanol, 3 - decane, 4 - dodecane, 5 - tetradecane, 6 - polychlorinated biphenyl PCB14, 7 - octadecane, 8 - diethylhexyl phthalate. It is obvious from Fig. 10 that using the presented protocol leads to almost total removal of added hydrophobic contaminants (compounds 3-8). The decrease of signal intensity of the mass spectrometer detector after filtration is greater than 100 for all these contaminants.

### Example 5

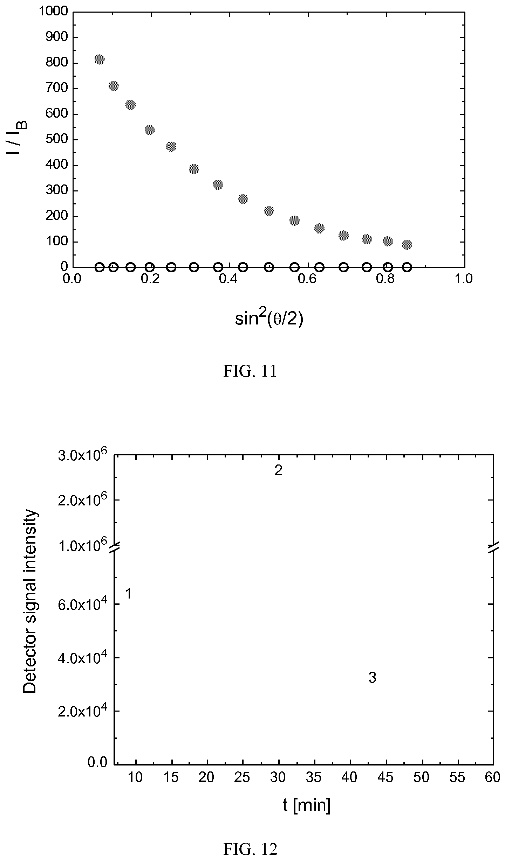

The procedure was the same as in Example 4. Tetrahydrofuran (THF) was used instead of ethanol. THF was intentionally contaminated with the following contaminants: decane (concentration 0.00151%), octadecane (concentration 0.00155%) and diethylhexyl phthalate (concentration 0.00150%). Overal concentration of contaminants 0.00456%. The contaminated THF was subsequently mixed with water in a ratio of 1:5.66 (15 wt. % solution of THF in water). Subsequently, the procedure was the same as in Example 1. Fig. 11 documents by means of static laser light scattering measurements the presence of nanoparticles in the contaminated THF/water mixture and their removal by filtration through a nanoporous filter with an effective pore diameter of 0.2 µm at a filtration pressure of 507 kPa (5 atm). Figure 12 shows the results of measurements using GC-MS HS-SPME method focused on the chemical composition of the mixture before and after filtration, in particular on the presence of contaminants. List of identified compounds: 1 - decane, 2 - octadecane, 3 - diethylhexyl phthalate. It is obvious from Fig. 12 that using the presented protocol leads to almost total removal of added hydrophobic contaminants (compounds 1-3).

### Example 6

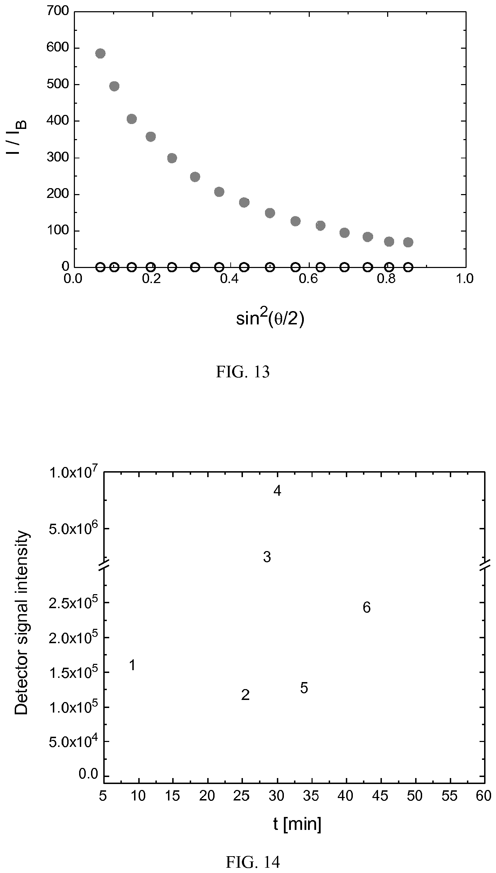

The procedure was the same as in Example 4. Dimethoxyethane was used instead of ethanol. Dimethoxyethane was intentionally contaminated with the following contaminants: decane (concentration 0.00150%), octadecane (concentration 0.00150%) and diethylhexyl phthalate (concentration 0.00150%). Overal concentration of contaminants 0.0045%. The contaminated dimethoxyethane was subsequently mixed with water in a ratio of 1:5.66 (15 wt. % solution of dimethoxyethane in water). Subsequently, the procedure was the same as in Example 1. Fig. 13 documents by means of static laser light scattering measurements the presence of nanoparticles in the contaminated dimethoxyethane/water mixture and their removal by filtration through a nanoporous filter with an effective pore diameter of 0.2 µm at a filtration pressure of 507 kPa (5 atm). Figure 14 shows the results of measurements using GC-MS HS-SPME method focused on the chemical composition of the mixture before and after filtration, in particular on the presence of contaminants. List of identified compounds: 1 - decane, 2 - hexadecane, 3 - methylheptadecane, 4 - octadecane, 5 - eicosane, 6 - diethylhexyl phthalate. It is obvious from Fig. 14 that using the presented protocol leads to almost total removal of added hydrophobic contaminants (compounds 1, 4 and 6) as well of contaminants contained in the original dimethoxyethane before its contamination (compounds 2, 3, 5 and broader spectrum of homologous hydrocarbons manifested in the elugram from 22nd to 37th minute).

### Example 7

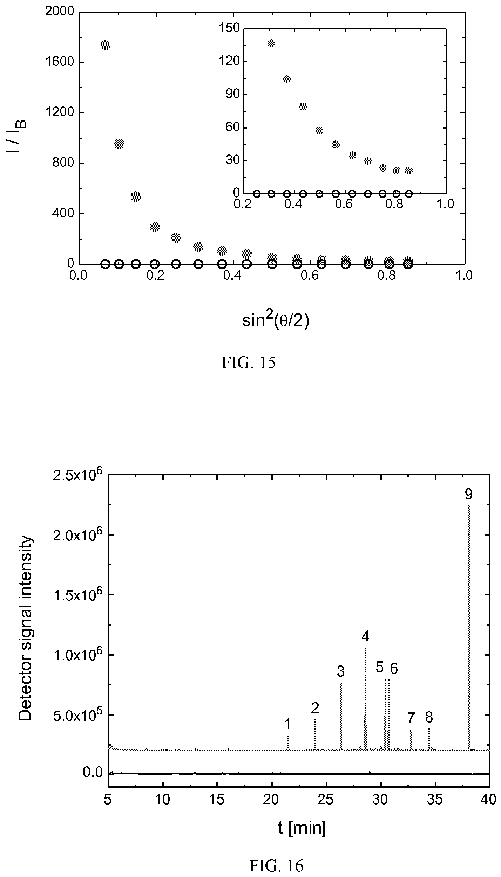

Example 7 documents the use of the present method not only for the purification of liquid organic compounds but also for the purification of solid organic compounds. The procedure was the same as in Example 1. Urea (15 wt. % solution of urea in water) was used instead of acetone. Fig. 15 documents, by means of static laser light scattering measurements, the presence of nanoparticles in the aqueous solution of urea and their removal by filtration through nanoporous filter with an effective pore diameter of 0.2 µm at a filtration pressure of 507 kPa (5 atm). Figure 16 shows the results of measurements using GC-MS HS-SPME method focused on the chemical composition of the mixture before and after filtration, in particular on the presence of contaminants. List of identified compounds: 1 - tetradecane , 2 - pentadecane, 3 - hexadecane, 4 - heptadecane, 5 - butyl dodecanoate , 6 - octadecane, 7 - nonadecane, 8 - butyl tetradecanoate, 9 - butyl hexadecanoate. It is obvious from Fig. 16 that using the presented protocol leads to almost total removal of hydrophobic contaminants (compounds 1-9).

### Example 8

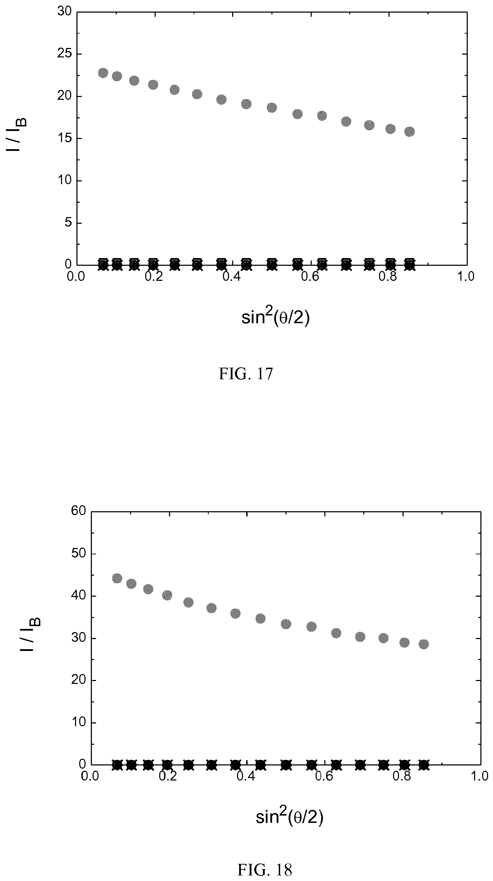

The procedure was the same as in Example 3. Fig. 17 documents by means of static laser light scattering measurements the presence of nanoparticles in TBA/water mixture and their removal by filtration through nanoporous filters with different pore diameters at a filtration pressure of 507 kPa (5 atm). It is obvious from Fig. 17 that using the presented protocol leads to almost total removal of hydrophobic contaminants using nanoporous filters with effective pore diameters of 0.45 µm (□), 0.2 µm (∘), 0.1 µm (+), and 0.02 µm (×).

### Example 9

The procedure was the same as in Example 1. Ethanol was used instead of acetone. Fig. 18 documents by means of static laser light scattering measurements the presence of nanoparticles in the ethanol/water mixture and their removal by filtration through 0.2 µm nanoporous filters at different pressures. It is obvious from Fig. 18 that using the presented protocol leads to almost total removal of hydrophobic contaminants using filtration pressures of 10.1 kPa (0.1 atm, □), 101 kPa (1 atm, ∘), 1.01 MPa (10 atm, +), and 10.1 MPa (100 atm, ×).

### Example 10

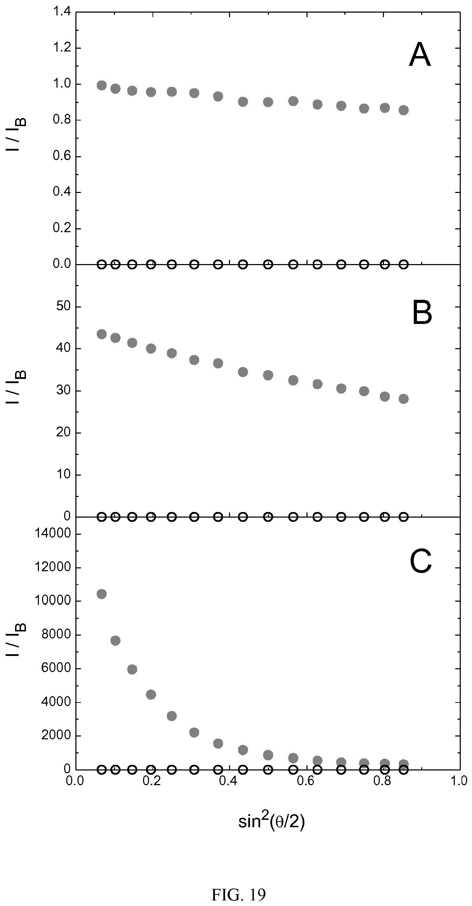

The procedure was the same as in Example 4. Instead of the mixture of contaminants, octadecane at concentrations of 0.00050% (A), 0.00564% (B) and 0.12562% (C) was used as contaminant. Fig. 19 documents by means of static laser light scattering measurements the presence of nanoparticles in the ethanol/water mixture and their removal by filtration through a 0.2 µm nanoporous filter at a filtration pressure of 507 kPa (5 atm). It is obvious from Fig. 19 that using the presented protocol leads to almost total removal of the hydrophobic contaminant at its concentration in the given purified water-soluble compound (ethanol) in the range from 0.00050% to 0.12%.

The applicability of the method in terms of the concentration range of the hydrophobic contaminant depends on the type of purified compound and the type of contaminant, and this range can be up to 0.0001% - 0.2%.

It is also possible to determine from Fig. 19A the final content of the contaminant after filtration. The decrease of scattering signal after filtration is at least 100-fold which corresponds to at least 10-fold decrease of contaminant concentration, namely from the initial value of 0.00050% to the value of 0.00005% (0.5 ppm).

### Example 11

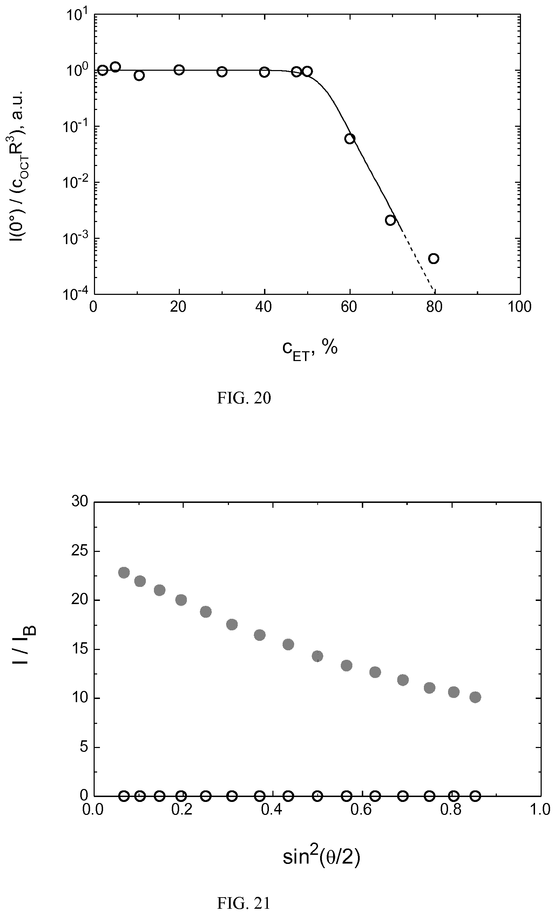

The procedure was the same as in Example 1. Ethanol of p.a. grade was used instead of acetone. Ethanol of p.a. grade was intentionally contaminated with octadecane (concentration 0.01501%). The contaminated ethanol was subsequently mixed with water in various proportions and nanosegregation of the contaminant (octadecane) was monitored by laser light scattering measurements in relation to the mixing ratio or to the concentration of ethanol in water expressed in wt. %. Fig. 20 shows the dependence of intensity of nanosegregation of the contaminant as a function of concentration of the organic liquid (ethanol) in the mixture with water.

Intensity of nanosegregation is quantified by intensity 1(0°) of scattering from the nanoparticles in the zero angle normalized to the concentration of octadecane in the ethanol/water mixture c_{OCT} and to the third power of the nanoparticles radius R³. In this manner the normalized intensity is proportional to the ratio of concentrations of segregated octadecane c_{sOCT} to the total concentration of octadecane in the ethanol/water mixture c_{OCT}. Fig. 20 shows that the effect of nanosegregation is present in a comparable intensity in the concentration range of ethanol from 1wt.% to 50 wt.%, while with increasing concentration of ethanol above 50 wt.% the effect of nanosegregation quickly ceases. In the concentration range of ethanol from 90% to 100% the effect of nanosegregation is zero. While Example 11 documents the concentration range of ethanol in the aqueous mixture where the effect of nanosegregation of hydrophobic contaminants is present, the following Examples 12 and 13 document the exploitability of this nanosegregation, i.e. the complete filterability of nanoparticles/nanodroplets of the segregated contaminant in the extremes of this interval, namely for 2 wt.% solution of ethanol in water and for 50 wt.% solution of ethanol in water.

### Example 12

The procedure was the same as in Example 1. Ethanol was used instead of acetone. Ethanol of p.a. grade was intentionally contaminated with octadecane (concentration 0.01501%). The contaminated ethanol was subsequently mixed with water in a ratio of 1:50 (2 wt. % solution of ethanol in water). Fig. 21 documents by means of static laser light scattering measurements the presence of nanoparticles in the ethanol/water mixture and their removal by filtration through a nanoporous filter with an effective pore diameter of 0.2 µm at a filtration pressure of 507 kPa (5 atm). It is obvious from Fig. 21 that using the presented protocol leads to almost total removal of hydrophobic contaminants.

### Example 13

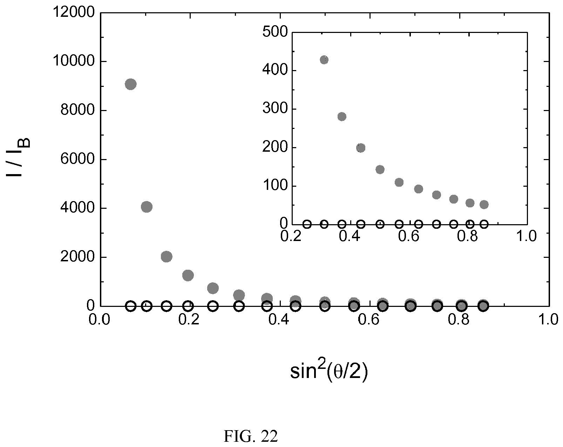

The procedure was the same as in Example 12. The contaminated ethanol was mixed with water in a ratio of 1:2 (50 wt. % solution of ethanol in water) instead of 1:50 ratio. Fig. 22 documents by means of static laser light scattering measurements the presence of nanoparticles in the ethanol/water mixture and their removal by filtration through a nanoporous filter with an effective pore diameter of 0.2 µm at a filtration pressure of 507 kPa (5 atm). It is obvious from Fig. 22 that using the presented protocol leads to almost total removal of hydrophobic contaminants.

### Industrial applicability

The application area of the given method is very wide - from the food industry through the pharmaceutical industry to pure chemicals for special and scientific applications.

## Claims

1. A method for purification of organic water-soluble compounds with solubility in water at least 1% from hydrophobic contaminants **characterized in that** the method includes the steps:
a) mixing a solid or liquid organic water-soluble compound containing hydrophobic contaminants with water, segregation of contaminants into nanoparticles/nanodroplets containing up to 99.9% of the contaminants originally present in the organic water-soluble compound, whereby the content of the organic water-soluble liquid compound containing hydrophobic contaminants in the mixture with water is from 1% to 50 wt.%, the content of the organic water-soluble solid compound containing hydrophobic contaminants in the mixture with water is from 1 wt.% to a value at which the given solid compound is still soluble in water within the solubility range in water for this compound,
b) removal of the nanoparticles/nanodroplets comprising hydrophobic contaminants from the water solution of solid or liquid organic compound using chemically resistant nanoporous filters with effective pore sizes from 5 nm to 500 nm and filtration pressures from 10.1 kPa to 1.01 MPa, whereby the formed nanoparticles/nanodroplets are substantially mechanically firm and stable in shape to be removable by filtration without necessity of addition of any additives as stabilizers of nanoparticles/nanodroplets and subsequent contamination of the mixture with these additives;
c) subsequent separation of water from the purified organic water-soluble compound by evaporation or distillation.

2. The method according to claim 1 **characterized in that** the hydrophobic contaminants are alkanes, polychlorinated biphenyls, polycyclic aromatic hydrocarbons, phthalates.

3. The method according to claims 1 and 2 **characterized in that** the alkanes are decane, dodecane, tetradecane, hexadecane, octadecane.

4. The method according to claims 1 and 2 **characterized in that** the polychlorinated biphenyls are polychlorinated biphenyl 14.

5. The method according to claims 1 and 2 **characterized in that** the polycyclic aromatic hydrocarbons are benzo(a)pyrene, naphthol, biphenyl.

6. The method according to claims 1 and 2 **characterized in that** the phthalates are diethylhexyl phthalate.

7. The method according to claim 1 **characterized in that** the organic water-soluble compounds are alcohols, acetone, tetrahydrofuran, dimethoxyethane, acetic acid, urea.

8. The method according to claims 1 and 7 **characterized in that** the alcohols are ethanol, tert-butyl alcohol.

9. The method according to claims 1-8 **characterized in that** the content of the organic water-soluble liquid compound containing hydrophobic contaminants in the mixture with water is from 10% to 25 wt.%.

10. The method according to claims 1-8 **characterized in that** the content of the organic water-soluble solid compound containing hydrophobic contaminants in the mixture with water is from 50% to 100% of the saturation concentration for the given particular compound.

11. The method according to claims 1-10 **characterized in that** the mixing of the organic water-soluble compound with water leading to the formation of filterable nanoparticles/nanodroplets is performed at temperatures at which the given mixture is in the liquid phase.

12. The method according to claims 1-11 **characterized in that** the total content of the contaminants in the organic water-soluble compound is from 0.0005% to 0.1%.

13. The method according to claims 1-11 **characterized in that** the total content of the contaminants in the organic water-soluble compound is from 0.0001% to 0.2%.

14. The method according to claims 1 - 13 **characterized in that** the effective pore size of the nanoporous filter is from 100 nm to 300 nm.

15. The method according to claims 1-14 **characterized in that** the applied filtration pressure is from 50.7 kPa to 304.0 kPa.

16. The method according to claims 1-15 **characterized in that** the content of the hydrophobic contaminant in the water-soluble organic compound is reduced after purification to a value of less than 5.0 ppm.

17. The method according to claims 1-16 **characterized in that** the content of the hydrophobic contaminant in the water-soluble organic compound is reduced after purification to a value of less than 0.5 ppm.

## Patentansprüche

1. Verfahren zur Reinigung von organischen wasserlöslichen Verbindungen mit einer Löslichkeit in Wasser von mindestens 1% von hydrophoben Kontaminanten, **dadurch gekennzeichnet, dass** das Verfahren die folgenden Schritte umfasst:
a) Mischung einer festen oder flüssigen organischen wasserlöslichen Verbindung, die hydrophobe Kontaminanten enthält, mit Wasser, Segregation der Kontaminanten in Nanopartikel/Nanotröpfchen, die bis zu 99,9 % der Kontaminanten, die ursprünglich in der organischen wasserlöslichen Verbindung vorhanden waren, enthalten, wobei der Gehalt an organischer wasserlöslicher flüssiger Verbindung, die hydrophobe Kontaminanten enthält, in der Mischung mit Wasser von 1% bis 50 Gew.-% beträgt, der Gehalt an organischer wasserlöslicher fester Verbindung, die hydrophobe Kontaminanten enthält, in der Mischung mit Wasser beträgt von 1 Gew.-% bis zu einem Wert, bei dem die gegebene feste Verbindung noch in Wasser löslich ist innerhalb des Löslichkeitsbereichs in Wasser für diese Verbindung,
b) Entfernung von Nanopartikeln/Nanotröpfchen, die aus hydrophoben Kontaminanten bestehen, aus der Wasserlösung der festen oder flüssigen organischen Verbindung unter Verwendung von chemisch resistenten nanoporösen Filtern mit effektiven Porengrößen von 5 nm bis 500 nm und Filtrationsdrücken von 10.1 kPa bis 1.01 MPa, wobei die gebildeten Nanopartikel/Nanotröpfchen ausreichend mechanisch fest und formstabil sind, um durch Filtration entfernt werden zu können, ohne die Notwendigkeit der Zugabe von Additiven als Stabilisatoren von Nanopartikeln/Nanotröpfchen und eine anschließende Verunreinigung der Mischung mit diesen Additiven;
c) anschließende Abtrennung des Wassers von der gereinigten organischen wasserlöslichen Verbindung durch Verdunstung oder Destillation.

2. Verfahren nach Anspruch 1, **dadurch gekennzeichnet, dass** die hydrophoben Kontaminanten Alkane, polychlorierte Biphenyle, polyzyklische aromatische Kohlenwasserstoffe, Phthalate sind.

3. Verfahren nach den Ansprüchen 1 und 2, **dadurch gekennzeichnet, dass** die Alkane Decan, Dodecan, Tetradecan, Hexadecan, Octadecan sind.

4. Verfahren nach den Ansprüchen 1 und 2, **dadurch gekennzeichnet, dass** die polychlorierten Biphenyle polychloriertes Biphenyl 14 sind.

5. Verfahren nach den Ansprüchen 1 und 2, **dadurch gekennzeichnet, dass** die polyzyklischen aromatischen Kohlenwasserstoffe Benzo[a]pyren, Naphtol, Biphenyl sind.

6. Verfahren nach den Ansprüchen 1 und 2, **dadurch gekennzeichnet, dass** die Phthalate Diethylhexylphthalat sind.

7. Verfahren nach Anspruch 1, **dadurch gekennzeichnet, dass** die organischen wasserlöslichen Verbindungen Alkohole, Aceton, Tetrahydrofuran, Dimethoxyethan, Essigsäure, Harnstoff sind.

8. Verfahren nach den Ansprüchen 1 und 7, **dadurch gekennzeichnet, dass** die Alkohole Ethanol, tert-Butylalkohol sind.

9. Verfahren nach den Ansprüchen 1 bis 8, **dadurch gekennzeichnet, dass** der Gehalt an organischer wasserlöslicher flüssiger Verbindung, die hydrophobe Kontaminanten enthält, in der Mischung mit Wasser von 10 % bis 25 Gew.-% beträgt.

10. Verfahren nach den Ansprüchen 1 bis 8, **dadurch gekennzeichnet, dass** der Gehalt an organischer wasserlöslicher fester Verbindung, die hydrophobe Kontaminanten enthält, in der Mischung mit Wasser von 50% bis 100% der Sättigungskonzentration für die gegebene konkrete Verbindung beträgt.

11. Verfahren nach den Ansprüchen 1 bis 10, **dadurch gekennzeichnet, dass** das Mischen der organischen wasserlöslichen Verbindung mit Wasser, das zur Bildung von filtrierbaren Nanopartikeln/Nanotröpfchen führt, bei Temperaturen durchgeführt wird, bei denen sich die gegebene Mischung in der flüssigen Phase befindet.

12. Verfahren nach den Ansprüchen 1 bis 11, **dadurch gekennzeichnet, dass** der Gesamtgehalt der Kontaminanten in der organischen wasserlöslichen Verbindung 0.0005% bis 0.1% beträgt.

13. Verfahren nach den Ansprüchen 1 bis 11, **dadurch gekennzeichnet, dass** der Gesamtgehalt der Kontaminanten in der organischen wasserlöslichen Verbindung 0.0001% bis 0.2% beträgt.

14. Verfahren nach den Ansprüchen 1 bis 13, **dadurch gekennzeichnet, dass** die effektive Porengröße des nanoporösen Filters von 100 nm bis 300 nm beträgt.

15. Verfahren nach den Ansprüchen 1 bis 14, **dadurch gekennzeichnet, dass** der angewandte Filtrationsdruck von 50.7 kPa bis 304.0 kPa beträgt.

16. Verfahren nach den Ansprüchen 1 bis 15, **dadurch gekennzeichnet, dass** der Gehalt des hydrophoben Kontaminanten in der wasserlöslichen organischen Verbindung nach der Reinigung auf einen Wert von weniger als 5.0 ppm reduziert wird.

17. Verfahren nach den Ansprüchen 1 bis 16, **dadurch gekennzeichnet, dass** der Gehalt des hydrophoben Kontaminanten in der wasserlöslichen organischen Verbindung nach der Reinigung auf einen Wert von weniger als 0.5 ppm reduziert wird.

## Revendications

1. Un procédé pour la purification de composés organiques solubles dans l'eau dont la solubilité dans l'eau est d'au moins 1 % à partir de contaminants hydrophobes, **caractérisé par le fait que** ledit procédé comprends les étapes suivantes :
a) mélange d'un composé organique soluble dans l'eau, solide ou liquide, contenant des contaminants hydrophobes, avec de l'eau, ségrégation des contaminants en nanoparticules/nanodroplets contenant jusqu'à 99,9 % des contaminants présents à l'origine dans le composé organique soluble dans l'eau, où le contenu du composé liquide organique soluble dans l'eau contenant des contaminants hydrophobes dans le mélange avec l'eau est compris entre 1 % et 50 W%, et le contenu du composé solide organique soluble dans l'eau contenant des contaminants hydrophobes dans le mélange avec l'eau est compris entre 1 W% et une valeur à laquelle le composé solide concerné est encore soluble dans l'eau, dans la gamme de solubilité dans l'eau pour ledit composé,
b) élimination des nanoparticules/nanodroplets contenant des contaminants hydrophobes de la solution aqueuse du composé organique solide ou liquide à l'aide de filtres nanoporeux chimiquement résistants avec des dimensions des pores effectives de 5 nm à 500 nm et pression de filtration de 10,1 kPa à 1,01 MPa, où les nanoparticules/nanodroplets formées sont substantiellement rigides mécaniquement et stables dans leur forme pour pouvoir être éliminés par filtration sans nécessité d'ajouter des additifs pour stabiliser les nanoparticules/nanodroplets et de contaminer ultérieurement le mélange avec lesdits additifs,
c) séparation successive de l'eau du composé organique purifié soluble dans l'eau par évaporation ou distillation.

2. Procédé selon la revendication 1 **caractérisé par le fait que** les contaminants hydrophobes sont des alcanes, biphényles polychlorés, hydrocarbures aromatiques polycycliques, phtalates.

3. Procédé selon les revendications 1 et 2 **caractérisé par le fait que** les alcanes sont des décanes, dodécanes, tétradécanes, hexadécanes, octadécanes.

4. Procédé selon les revendications 1 et 2 **caractérisé par le fait que** les biphényles polychlorés sont du biphényle polychloré 14.

5. Procédé selon les revendications 1 et 2 **caractérisé par le fait que** les hydrocarbures aromatiques polycycliques sont du benzo(a)pyrène, naphtol, biphényle.

6. Procédé selon les revendications 1 et 2 **caractérisé par le fait que** les phtalates sont du phthalate de diéthylhexyle.

7. Procédé selon la revendication 1 **caractérisé par le fait que** les composants organiques solubles dans l'eau est formée par des alcools, acétone, tétrahydrofurane, diméthoxyethane, acide acétique, urée.

8. Procédé selon les revendications 1 et 7 **caractérisé par le fait que** les alcools sont de l'éthanol, alcool tert-butylique.

9. Procédé selon les revendications 1 - 8 **caractérisé par le fait que** le contenu du composant organique liquide soluble dans l'eau contenant des contaminants hydrophobes dans le mélange avec l'eau est compris dans une gamme entre 10 % et 25 W%.

10. Procédé selon les revendications 1 - 8 **caractérisé par le fait que** le contenu du composant organique solide soluble dans l'eau contenant des contaminants hydrophobes dans le mélange avec de l'eau est compris dans une gamme entre 50 % et 100 % de la concentration de saturation pour le composant particulier concerné.

11. Procédé selon les revendications 1 - 10 **caractérisé par le fait que** le mélange du composant organique soluble dans l'eau avec de l'eau, causant la formation de nanoparticules/nanodroplets filtrables, est réalisé à des températures auxquelles ledit mélange est en phase liquide.

12. Procédé selon les revendications 1 - 11 **caractérisé par le fait que** le contenu total des contaminants dans le composant organique soluble dans l'eau est compris entre 0,0005 % et 0,1 %.

13. Procédé selon les revendications 1 - 11 **caractérisé par le fait que** le contenu total des contaminants dans le composant organique soluble dans l'eau est compris entre 0,0001 % et 0,2 %.

14. Procédé selon les revendications 1 - 13 **caractérisé par le fait que** les dimensions des pores effectives du filtre nanoporeux sont comprises entre 100 nm et 300 nm.

15. Procédé selon les revendications 1 - 14 **caractérisé par le fait que** la pression de filtration appliquée est comprise entre 50,7 kPa et 304,0 kPa.

16. Procédé selon les revendications 1 - 15 **caractérisé par le fait que** le contenu du contaminant hydrophobe dans le composant organique soluble dans l'eau est réduit après la purification à une valeur de moins de 5,0 ppm.

17. Procédé selon les revendications 1 - 16 **caractérisé par le fait que** le contenu du contaminant hydrophobe dans le composant organique soluble dans l'eau est réduit après la purification à une valeur de moins de 0,5 ppm.
